# EUROPEAN PATENT SPECIFICATION

(11) **EP 1 349 568 B1**
(45) Date of publication and mention of the grant of the patent: **16.09.2009**
(21) Application number: 01999384.9
(22) Date of filing: 04.12.2001
(51) Int. Cl.: A61K 39/00, A61K 39/38, G01N 33/53

(54) **IMMUNOMODULATORY CONSTRUCTS AND THEIR USES**
IMMUNMODULATORISCHE KONSTRUKTIONEN UND IHRE VERWENDUNG
CONSTRUCTIONS IMMUNOMODULATRICES ET UTILISATIONS ASSOCIEES

(30) Priority: 04.12.2000 US 251243 P
(43) Date of publication of application: 08.10.2003
(73) Proprietor: AUCKLAND UNISERVICES LIMITED, Auckland (NZ)
(72) Inventor: FRASER, John David, Meadowbank Auckland (NZ); NICHOLSON, Melissa, Joy, Kingsland, Auckland (NZ)
(74) Representative: Cornish, Kristina Victoria Joy
(86) International application number: PCT/NZ2001/000267
(87) International publication number: WO 2002/045739

(56) References cited:
- WO-A-98/24910
- WO-A-98/26747
- US-A- 5 968 514
- LEDER LUKAS ET AL: "A mutational analysis of the binding of staphylococcal enterotoxins B and C3 to the T cell receptor beta chain and major histocompatibility complex class II" JOURNAL OF EXPERIMENTAL MEDICINE, vol. 187, no. 6, 16 March 1998 (1998-03-16), pages 823-833, XP002903430 ISSN: 0022-1007
- LI HONGMIN ET AL: "Three-dimensional structure of the complex between a T cell receptor beta chain and the superantigen staphylococcal enterotoxin B" IMMUNITY, vol. 9, no. 6, December 1998 (1998-12), pages 807-816, XP002314864 ISSN: 1074-7613
- FRASER JOHN ET AL: "Superantigens: Powerful modifiers of the immune system" MOLECULAR MEDICINE TODAY, vol. 6, no. 3, March 2000 (2000-03), pages 125-132, XP002314865 ISSN: 1357-4310
- PROFT T. AND FRASER J.: 'Superantigens: Just like peptides only different' JOURNAL OF EXPERIMENTAL MEDICINE vol. 187, no. 6, 1998, pages 819 - 821, XP002903301

## Description

### TECHNICAL FIELD

This invention relates to immunomodulatory constructs and their use. In particular, it relates to constructs which target antigen-presenting-cells for the purpose of enhancing or suppressing a host immune response, and to methods of enhancing antigenicity of compounds.

### BACKGROUND ART

Professional antigen-presenting-cells (APC) are essential to initiate a primary immune response in a non-immune, naive animal. The most important APC is the Dendritic Cell (DC), which is found as an interdigitating cell at all regions of the body, at an interface with the environment (i.e. skin and mucosal surfaces such as the lung, airways, nasal passage etc). Antigens presented by DCs are profoundly immunogenic. One important phenotypic marker of the DC is a very high level of surface MHC class II expression. Activated DCs migrate to secondary lymph nodes to "prime" both CD4 and CD8 T cells which proceed as antigen activated effector cells, to proliferate, produce cytokines and regulate the humoral response of B-lymphocytes. Thus, antigen presentation by DC appears to be the obligate first step in any adaptive immune response. Other APCs such as macrophages and B-cells appear to be important in later, secondary responses and by themselves are not effective in the initial priming of a response. Thus the DC is generally regarded as the most important cell to target for enhancement of immune responses.

The targeting of antigens to DC can however be problematic. For example, many peptides by themselves are poorly antigenic and immunogenic because they are not efficiently delivered to APC in vivo. They are equally not taken up by APC very efficiently and do not elicit the second signals required for efficient antigen presentation.

Superantigens are a family of semi-conserved bacterial proteins that target the immune system by binding simultaneously to the T cell Receptor (TcR) via the Vβ domain on T lymphocytes and MHC class II molecules expressed on APC including dendritic cells.

Superantigens (SAgs) are the most potent immune mitogens known and activate large numbers of T cells at femto-attomolar concentrations (10⁻¹⁵-10⁻¹⁸M). They cause significant toxicity due to the massive systemic cytokine release by T cells. There are currently 19 members of the staphylococcal and streptococcal superantigen family.

Terman (WO 98/26747) discloses therapeutic compositions employing superantigens. It is suggested that superantigens, in conjunction with one or more additional immunotherapeutic antigens, may be used to either induce a therapeutic immune response directed against a target or to inhibit a disease-causing immune response. Terman further describes the formation of immunotherapeutic antigen-superantigen polymers. Such polymers include those were the superantigen component is coupled to a peptide antigen by a secondary amine linkage. However, there is no teaching or suggestion by Terman that the superantigen component be one from which the TcR binding function has been wholly or partly ablated. Indeed, there is no recognition that a TcR binding is not essential to activation of APCs and to stimulation of an immune response against the antigenic component of the polymer.

Thus, wild-type SAgs, or modified SAgs which retain the ability to bind to TcR, are of little use because they themselves elicit massive, indiscriminate T cell responses by binding to the TcR. This TcR cross-linking appears to be the major cause of their toxicity ¹².

There exists a need therefore for improved immunomodulators which exploit the unique features of DC targeting and activation of SAgs to deliver and enhance the T cell recognition of antigens such as peptides that are normally non-immunogenic or have low immunogenicity, yet are efficacious and have low toxicity.

It is an object of the present invention to overcome or ameliorate at least some of the disadvantages of the prior art, or to provide a useful alternative.

### SUMMARY OF THE INVENTION

According,to a first aspect there is provided an immunomodulator which comprises a superantigen coupled to an antigen, wherein said superantigen includes a Class II MHC binding site and a T-cell receptor binding site of a superantigen, the T-cell receptor binding site having one or more mutations that remove or minimise TCR binding compared to the wildtype T-cell receptor binding site, and wherein in use the immunomodulator can bind Class II MHC molecules.

There is provided an immunomodulator which comprises an antigen-presenting cell (APC) targeting molecule coupled to an immunomodulatory antigen, wherein said APC-targeting molecule is a molecule which is structurally a superantigen but for a disrupted T-cell receptor binding site such that the molecule has little or no ability to activate T-cells

Preferably the superantigen is derived from *Staphylococcus aureus* and/or *Streptococcus pyogenes*. Particularly preferred is a superantigen derived from SPE-C and the preferred truncation involves deletion of residues 22-90 from the wild-type SPE-C sequence. However it will be clear to those skilled in the art that other SAgs which have a similar or otherwise known TcR binding region of the molecule may also be advantageously used, for example SMEZ, SEA and the like.

The T-cell receptor binding site, or at least a part thereof, of the superantigen can also been modified by substitution or addition, to remove or minimise TcR binding. An example of such a targeting molecule is SPEC-Y15A R181Q of the present invention.

A particularly preferred intermediate in the generation of the immunomodulator is Y15A.C27S.N79C.

Preferably the coupling between the superantigen and the antigen will be reversible. However, it will be understood from the following description that what is preferably required is that the superantigen is capable of releasing the immunomodulatory antigen so that it is correctly presented by the APC. Thus, it would also be clear that the release of the immunomodulatory antigen from the immunomodulator may be achieved by intracellular or intralysosomal enzymatic cleavage. This process may be assisted by introducing the appropriate proteolytic site into the coupling region of the immunomodulator. The release may also be achieved by chemical means, which includes redox reactions involving disulphides and free sulphydryl groups. This process may also be assisted by introducing into the coupling region certain amino acid residues, eg. cysteine.

Preferably the antigen is a protein, a polypeptide and/or a peptide however similar principles may be applied to antigens which are non-proteinaceous, for example nucleic acids or carbohydrates.

The antigen may be entirely non-immunogenic when not coupled to the superantigen but the immunomodulators of the present invention may also incorporate antigens which are immunogenic, in order to improve their efficacy. Thus the present invention is equally applicable to for example to new vaccines as it is to those which are already known and used but which can be improved by means of the immunomodulators of the present invention.

According to a second aspect there is provided a pharmaceutical composition comprising an immunomodulator according to the present invention and a pharmaceutically acceptable carrier, adjuvant, excipient and/or solvent.

According to a third aspect there is provided a vaccine comprising an immunomodulator according to the present invention.

According to a fourth aspect there is provided an immunomodulator or of a pharmaceutical composition or vaccive according to the present invention, for use in the treatment of a disorder which requires the induction or stimulation of the immune system

Preferably the disorder is selected from the group consisting of bacterial, viral, fungal or parasitic infection, autoimmunity, allergy and/or pre-neoplastic or neoplastic Transformation.

According to a fifth aspect there is provided the use of an immunomodulator according to the first or the second aspect for the preparation of a medicament for the therapeutic or prophylactic treatment of a disorder which requires the induction or stimulation of the immune system.

The preferred disorder is selected from the group consisting of bacterial, viral, fungal or parasitic infection, autoimmunity, allergy and/or pre-neoplastic or neoplastic transformation.

According to a sixth aspect there is provided a method of preparing an immunomodulator of the first aspect comprising the steps of:
a introducing a modification and/or a deletion into the T-cell binding site of a superantigen, and
b coupling thereto an immunomodulatory antigen.

Preferably the superantigen is selected from the group of SPE-C, SMEZ and SEA and more preferred are the antigen-presenting cell (APC) targeting molecules SPE-C Y15A. R181Q or SPEC (-20-90). Even more preferred is SPEC-Y15A.C27S.N79C.R181Q

It will be understood however that more than one superantigen may be employed and that a combination of immunomodulators may be used in any treatment.

### BRIEF DESCRIPTION OF THE FIGURES

**Figure 1****.** Antigenicity of SAG:PCC conjugate
**Figure 2****.** Immunogenicity of SPEC:PCC conjugate
**Figure 3****.** Proliferation of 5C.C7 LN cells to SPEC-CytC vs MHC-/-SPEC-CytC and free CytC peptide in vitro

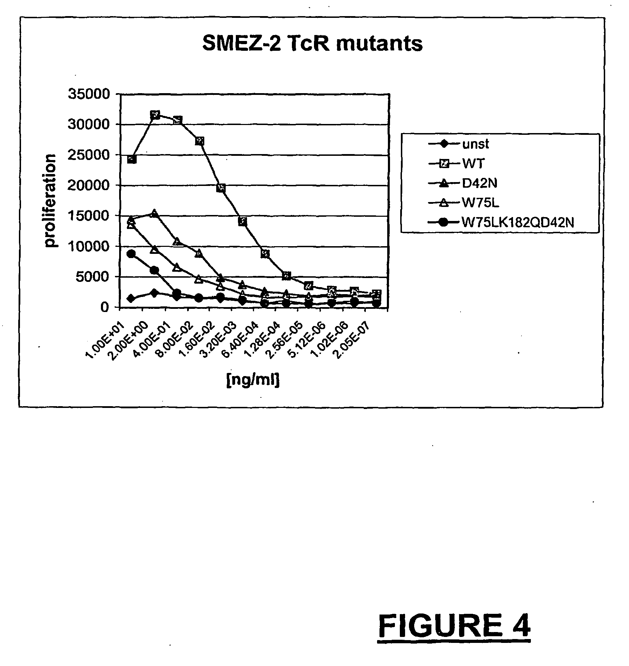

**Figure 4****.** Proliferative responses of SMEZ TcR mutants

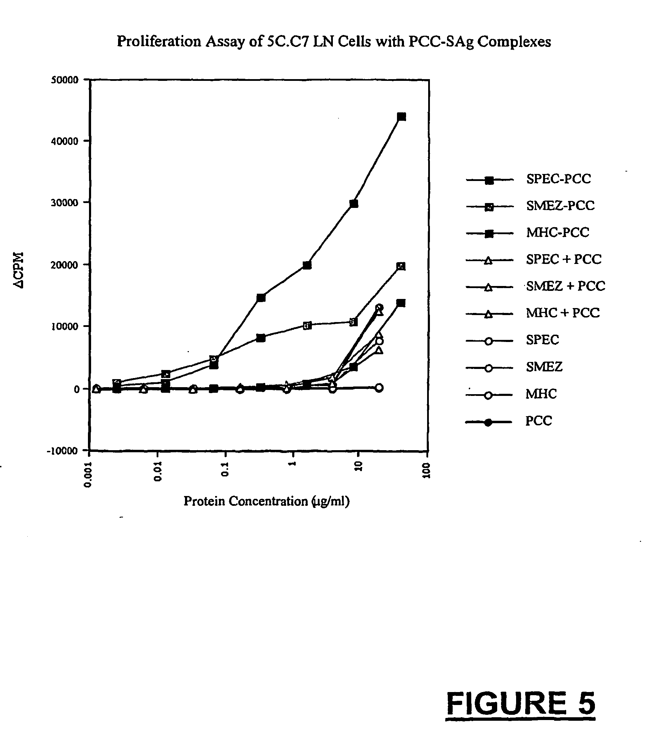

**Figure 5****.** Proliferative responses of 5C.C7 LN Cells with PCC-SAg Complexes. (Legend: The red line indicates the proliferative response to PCC protein alone. The blue square line shows that the response to PCC-SPEC is 100-fold more antigenic than the unconjugated PCC protein. The green square line is the response to PCC-SMEZ and is approximately 80 fold more antigenic than to unconjugated PCC protein. The black square shows the response to PCC conjugated to SPEC defective in MHC class II binding is no greater than the response to unconjugated PCC protein. The triangles represent the proliferative response of T cells to SAG and PCC together as a mixture but not conjugated).

### DESCRIPTION OF THE PREFERRED EMBODIMENT

The present invention is based at least in part on an unexpected observation that a molecule which mimics a superantigen but which lacks a fully functional TcR binding site can, when coupled to an immunomodulatory antigen, bind and activate APCs to a degree not previously known or suspected. Thus, such immunomodulatory constructs are effective in antigen presentation without the requirement to bind to the TcR. This is of particular relevance to moieties which have low or nonexistent immunogenicity, such as peptides, proteins, nucleic acids, whole viruses etc

The applications of this technology rely on the ability to generate a wide variety of immunomodulatory reagents that combine the delivery capacity and APC activating potential of the TcR ablated superantigens with the specificity of a coupled antigen.

A preferred use of this technique is to enhance responses to synthetic peptides as has been displayed herein with the PCC peptide. However, the antigen need not be a synthetic peptide, but could be a native or recombinant polypeptide, protein of even whole disabled virus. Further, the antigen need not be proteinaceous and may be a nucleic acid or carbohydrate antigen. Also, the present invention can be applied to antigens which are immunogenic, by improving immunogenicity or reducing the quantity of antigen required to induce an immune response

Peptides can be designed to be either stimulatory (i.e. generate agonist responses) or immunosuppressive (i.e. generate antagonist responses) to induce tolerance depending on the primary sequence of the peptide. This is useful in either promoting immunity for vaccination against pathogens such as viruses, bacteria and other micro-organisms, or for generating specific anti-tumour immunity using tumour specific peptides.

Antagonist responses induce T cell tolerance to antigen and might be useful to suppressing unwanted autoimmune reaction to self-antigens eg. proteins and/or nucleic acids, in the case of diseases such as multiple sclerosis, diabetes or rheumatoid arthritis.

Many autoimmune diseases have their basis in an auto-reactive T cell response to self antigens. Diseases such as rheumatoid arthritis, multiple sclerosis and diabetes mellitus are such examples.

The present invention will now be exemplified more particularly with reference to non-limiting examples.

### EXAMPLES

### Example 1: Cloning and expression of superantigen genes

Genes coding for individual wild-type superantigens were isolated and cloned directly from the DNA of isolates of *Staphylococcus aureus* or *Streptococcus pyogenes* using polymerase chain reaction (PCR) and oligonucleotides inferred from published sequences. All wild type sequences have been confirmed by DNA sequencing.

The methods used for isolation, cloning and sequencing are standard laboratory procedures and are described in for example Goshorn SC, Schlievert PM. 1988. Nucleotide sequence of streptococcal pyrogenic exotoxin type C. Infect Immun. 56(9):2518-20. Proft T, Moffatt SL, Berkahn CJ, Fraser JD. 1999. Identification and characterization of novel superantigens from Streptococcus pyogenes. J Exp Med. Jan 4;189(1):89-102, all incorporated herein by reference.

A summary of the SPE-C single domain molecule and its derivation is set out below, including the comparative proliferative response of human T cells.

### C-terminal Single Domain of SPE-C

(C-terminal single domain references the term "truncated SPE-C" and is a reference to the explicitly stated SPEC-(-20-90). The parenthesized numbers represent that part of the native SPE-C that has been deleted as outlined in the procedure below)
Vector: pGEX-3C (variation of pGEX-2T)
Host: DH5
Antibiotic resistance: Ampicillin
Restriction sites: 5' BamH1, 3' EcoR1

### Brief Expression protocol:

*. Grow overnight culture in LB-Amp at 37°C with shaking.
*. Dilute overnight culture 1:10 with pre-warmed LB-Amp.
*. Grow for another hour or until the absorbance at 600nm is 0.9.
*. Cool culture to 30°C.
*. Induce protein expression with 0.1 mM IPTG.
*. Incubate at 30°C with shaking for 4 - 5 hours.
*. Harvest cells and resuspend in 10 mls of GSH Buffer 1 (25 mM Tris.Cl pH 7.4 / 50 mM NaCl / 1 mM EDTA) for every 1 gram of pellet.
*. Sonicate to lyse cells and release soluble fusion protein.
*. Spin lysate to remove insoluble material.
*. Dialyse lysate overnight in GSH Buffer 1 to remove endogenous GSH (this step will increase yields but is not essential).
*. Purify GST-Fusion protein from bacterial proteins using GSH agarose affinity chromatography.
*. Cut purified fusion protein overnight with 3C protease at 4°C (NB to add DTT)
*. Dialyse cut fusion protein into 10 mM PO₄ pH 6.0 overnight.
*. Purify C-terminal Single Domain from GST using cation exchange chromatography (ie MonoS column - elute with pH gradient 6.0 - 7.0 over 20 column volumes)

### Sequence details:

Includes residues 1 - 21 of SPE-C, 4 amino acid linker which is the Factor X protease cleavage site, and then residues 91 - 208 of SPE-C.

### DNA sequence (Factor X sequence shown in gray):

GAC TCT AAG AAA GAC ATT TCG AAT GTT AAA AGT GAT TTA CTT TGC GCA TAC
ACT ATA ACT CCT ATC GAA GGT CGT ACG CCT GCT CAA AAT AAT AAA GTA AAT
CAT AAA TTA TTG GGA AAT CTA TTT ATT TCG GGA GAA TCT CAA CAG AAC TTA
AAT AAC AAG ATT ATT CTA GAA AAG GAT ACC GTA ACT TTC CAG GAA ATT GAC
TTT AAA ATC AGA AAA TAC CTT ATG GAT AAT TAT AAA ATT TAT GAC GCT ACT
TCT CCT TAT GTA AGC GGC AGA ATC GAA ATT GGC ACA AAA GAT GGA AAA CAT
GAG CAA ATA GAC TTA TTT GAC TCA CCA AAT GAA GGG ACT AGA TCA GAT ATT
TTT GCA AAA TAT AAA GAT AAT AGA ATT ATC AAT ATG AAG AAC TTT AGT CAT
TTC GAT ATT TAT CTT GAA AAA TAA

### Protein Parameters:

### Protein Sequence:

DSKKDISNVKSDLLCAYTITPIEGRTPAQNNKVNHKLLGNLFISG
ESQQNLNNKIILEKDTVTFQEIDFKIRKYLMDNYKIYDATSPYVS
GRIEIGTKDGKHEQIDLFDSPNEGTRSDIFAKYKDNRIINMKNFSH
FDIYLEK Stop

Molecular Weight: 16543
Theoretical pl: 7.02
Theoretical Extinction data (6M Guanidine-HCI/20mM phosphate, pH 6.5)
Assuming all cysteines are reduced:
Molar A280: 8960
A280/cm (1 mg/ml): 0.542

### Activity of C-terminal Domain SPE-C

### PBL stimulation assay:

Peripheral blood lymphocytes are isolated from blood using Hypaque-Ficoll. A 5 fold serial dilution of toxin in RPMI (complete) is set up in a 96 well plate. 1 x 10⁵ PBLs is added to each well containing varying concentrations of toxins. The plates are left to incubate for 3 days after which time [³H]Thymidine is added to each well to measure proliferation. The cells are harvested the next day and [³H]Thymidine incorporation is measured.

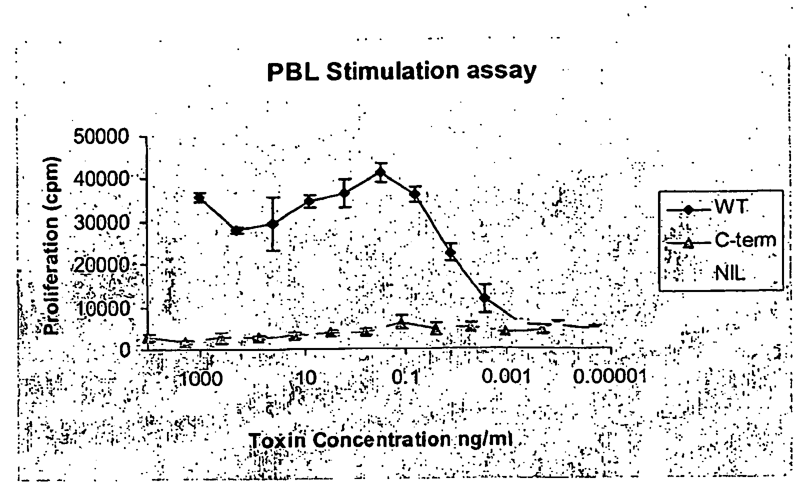

The above figure shows that the C-terminal domain SPE-C does not have stimulatory activity above background with human PBLs. This is most likely due to the fact that it cannot interact with the TcR on T cells or cross-link MHC on the cell surface of antigen presenting cells.

### Example 2: Ablation of TcR binding residues in superantigens

The gene from SPE-C was derived from a patient isolate of Streptococcus pyogenes by PCR using synthetic primers to the 5' and 3' end of the genes. These primer sequences were obtained from the published sequence of Goshorn SC, Schlievert PM. 1988. Nucleotide sequence of streptococcal pyrogenic exotoxin type C. Infect Immun. 56(9):2518-20. GenBank accession number M35514. Any other Streptococcus pyogenese isolate can also be used for this purpose.

Primers used to amplify the SPEC gene are listed in table 1 as SPEC-N-terminal and SPEC-C-terminal. The sequence was confirmed by DNA sequencing.

The full length SPE-C gene was sub-cloned into the expression vector pGEX-3T (Pharmacia) following manufacturers instructions which was used to transform the bacteria *E*. *coli* using standard procedures (Maniatis et al, ). Recombinant SPE-C fused to glutathione-S-transferase was purified from *E*. *coli* cultures using Glutathione Agarose affinity chromatography.

**Table 1: Primers used for amplification of the SPEC gene and introduction of mutations or truncations**

| | | |
|---|---|---|
| SPEC - N-terminal | CGGGATCCGACTCTCAAGAAAGACA | |
| SPEC - C-terminal | CTGAATTCTTATTTTTCAAGAT | |
| SPEC-Y15A | GATTTACTTTGTGCATACAC | GTGTATGCACAAAGTAAATC |
| SPEC-N79C | ATATTCTTTGTTCTCACA | TATAAGAAACAAGAGTGT |
| SPEC-Y15C | GATTTACTTTGTGCATACAC | GTGTATGCACAAAGTAAATC |
| SPEC-R181Q | GAAGGGACTCAATCAGATATTTTTGC | GACAAAATATCTGATTGAGTCCCTTC |
| SPEC-(-20-90) | ATCGAAGGTCGTACGCCTGCTCAAAATAATAAAG | ACGACCTTCGATAGGAGTTATAGTGTAT |
| SPEC-C27S | GATTATAAAGATTCCAGGGTAA | TTACCCTGGAATCTTTATAATC |

| | | |
|---|---|---|
| Sequential introduction of current mutations into SPE-C. **1. SPEC - C27S** To remove a naturally occurring cysteine that interferes with the coupling of antigen to the preferred site at N79C. **2. SPEC - C27S, N79C** To introduce the coupling point for antigen. This position was chosen from the crystal structure of SPE-C to be well exposed and to not interfere with MHC class II binding. **3. SPEC - C27S, N79C, Y15A** To destroy TcR binding **4. SPEC - C27S, N79C, R181Q** To further limit binding of T cell Receptors. | | |

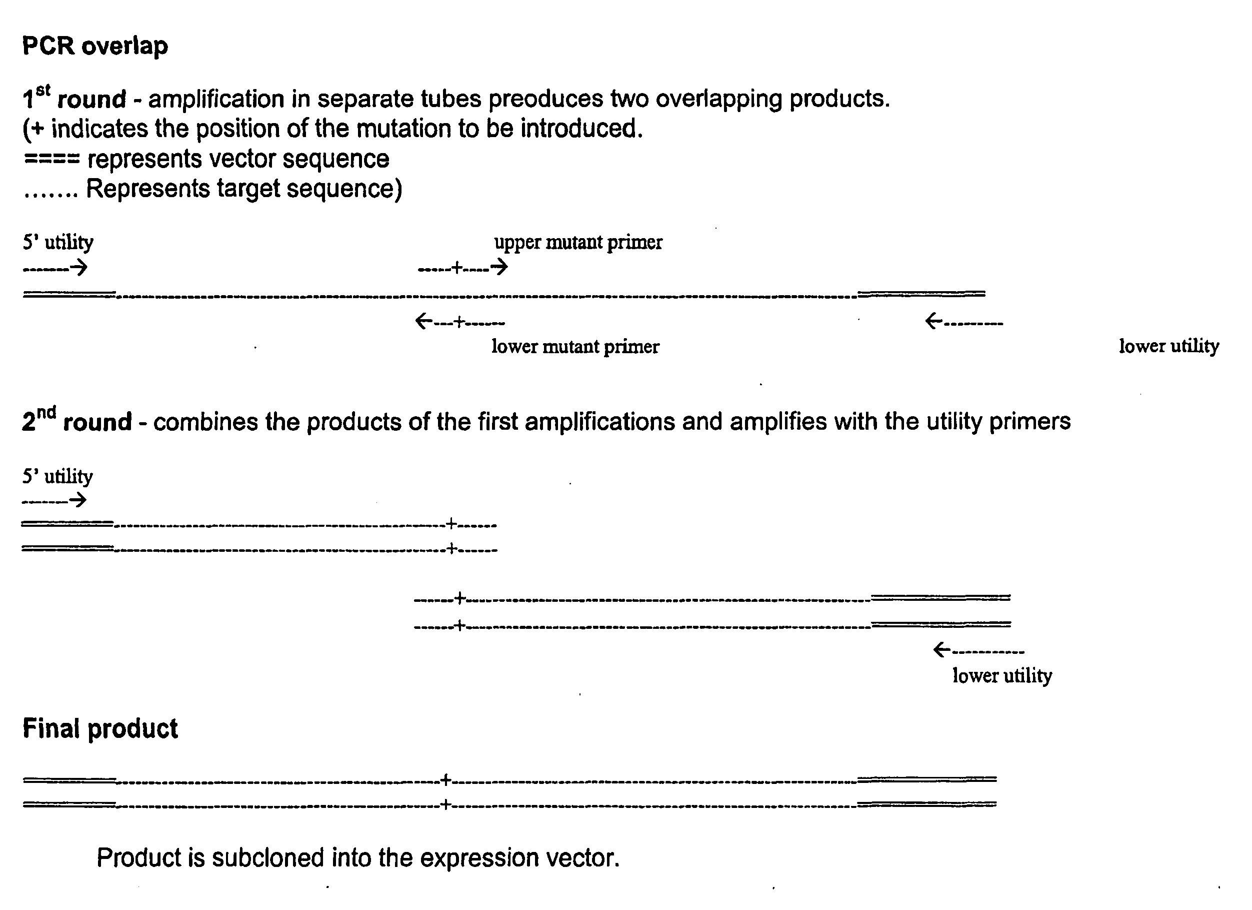

Glutathione-Agarose was manufactured according to previously published methods^{22,23}. Recombinant SPE-C protein was purified after cleavage of the fusion protein with trypsin by ion cation exchange chromatography according to the method described in reference 5 which is incorporated herein. Purified SPE-C was crystalised and the 3-D structure determined according to Roussel, 1997 (Ref 26), which is incorporated herein by reference.

Identification of amino acids in SPE-C that are important to TcR binding were determined by a combination of molecular modelling of the 3D crystal structure of SPE-C and comparison with known TcR binding residues of the related superantigen SEB.

### Rational mutagenesis of residues thought to be part of the TcR interface.

TcR binding residues were targeted by site-directed mutagenesis using the method of PCR overlap ²⁴.. The synthetic primers used to produce each mutation are described in the accompanying table of primers (Table 1). The process of introducing two mutations was performed sequentially as described in the accompanying diagrams describing the sequential introduction of successive mutations in SPE-C and the method of PCR overlap which is used to introduce said mutations.

The mutant form of SPE-C of the present invention was confirmed by automated DNA sequencing (Licor Inc. USA) then inserted into the pGEX expression vector between the BamH1 and EcoR1 restrictions sites according to the manufacturers description of the cloning site for this vector. A strain of *E. coli* DH5a was transformed with the recombinant vector and colonies expressing the pGEX fusion protein were isolated to grow up in large scale cultures for the purposes of protein purification.

To test the effects of mutations in the TcR binding site, recombinant proteins were added to cultures of human peripheral blood lymphocytes, isolated by standard techniques (for examples of techniques see Handbook Of Experimental Immunology, ed. D.M. Weir, Blackwell Scientific Publications), to determine what concentration of recombinant SPE-C was required to stimulate the proliferation of human T cells. Wild-type SPE-C normally stimulates human T cells at 50% of maximal proliferation at 0.2 pg/ml. Two residues were identified from these studies that when mutated, reduce T cell proliferation by 1,000,000 fold when compared to wild-type SPE-C. These residues are Y15 and R181. SPE-C molecules with these two mutations (SPEC-Y15A, R181Q) no longer stimulate human T cells

Amino acid residues in superantigens that are important to the interaction with T cell Receptor have been identified from the present mutational studies and those of others (Table 2 below). Loss of T cell activation is determined by *in vitro* T cell proliferation assays (see below) and compared to the activity of wild-type molecule. All mutants are also assessed for their ability to bind to MHC class II by a number of assays including direct binding to MHC class II expressing B cells as well as Biacore studies with soluble forms of both superantigen mutant and MHC class II.

3D crystal structures of the superantigens SEC3 bound to a murine T cell Receptor ^{4,13} provides the most complete information about the nature of superantigen/TcR interaction but is limited to those with SEC3-like activity. Most single point mutations result in only a small loss in superantigen activity due to only small reductions in binding affinity to the TcR. It is rare to find a single mutation that completely abrogates all mitogenic potential. Only SPE-C Y15A has been shown (Yamoaka et al, Infect. Immunol. 1998 66:5020 and McCormick et al, J. Immunol. 2000 165: 2306-2312) to cause more than a 1000-fold reduction in T cell responses to a superantigen.

The combined mutations producing SPE-C Y15A, R181Q of the present invention generates a form of SPE-C that has no detectable T cell activating potential.

By homology modelling of the 3D crystal structures of other SAgs important legions for binding to the TcR can be identified and corresponding mutants prepared and used to generate immunomodulators of the present invention.

### Example 3: T cell proliferation assay The T cell proliferation assay used was a standard technique described for example in REF 5, incorporated herein by reference

Purified recombinant mutant superantigens are incubated with freshly isolated human peripheral blood lymphocytes at varying dilutions in microtitre plates for 3 days. A fixed amount of ³H thymidine is added on the 3^{rd} day and the cells are harvested on day 4. The amount of ³H thymidine incorporated into the cellular DNA is measured by scintillation autography and is a direct measure of the degree of cell proliferation. Mutant superantigens are compared to wild-type superantigens. The proliferative potential of a given superantigen or mutant is expressed as the concentration required to induce 50% of its maximal stimulation (P_{50%}).

A fully ablated TcR binding negative superantigen is defined herein as one that displays less than about 0.0001% of proliferative activity of the wild-type superantigen (i.e. a 1 million-fold reduction in activity).

**Table 2. Amino acid residues implicated in TcR binding of known superantigens.**

| | Residues implicated in TcR binding sites | References |
|---|---|---|
| SEA | N25; P206, D207 | ^{5,14} |
| SEB | N23, Y90 | ¹² |
| SEC3 | G19, T20, N23, Y26, N60, Y90, V91, G102, K103, V104, G106, F176, Q210 | ^{13,4} |
| SEE | N23, S206, N207 | ^{5,14} |
| TSST | Tyr115, Glu132, His135, Ile140, His141 and Tyr144, Q136A | ^{15,16} |
| SPE-C | Y15*, R181* | Present invention |
| SMEZ-2 | D42N, W75L,Y77A, K182Q, S7A, N11A, D181A | Present invention |

| | | |
|---|---|---|
| Those in bold indicate mutations that decrease activity by more than 100-fold. * Mutation that totally ablates T cell responses | | |

Primary DNA sequences of the wild-type and the mutant form of SPE-C are detailed below:

### SPE-C wild type (from GenBank)

### Streptococcus pyogenes pyrogenic exotoxin C gene, 5' end cds

GACTCTAAGA AAGACATTTC GAATGTTAAA AGTGATTTAC TTTATGCATA CACTATAACT
CCTTATGATT ATAAAGATTG CAGGGTAAAT TTTTCAACGA CACACACATT AAACATTGAT
ACTCAAAAAT ATAGAGGGAA AGACTATTAT ATTAGTTCCG AAATGTCTTA TGAGGCCTCT
CAAAAATTTA AACGAGATGA TCATGTAGAT GTTTTTGGAT TATTTTATAT TCTTAATTCT
CACACCGGTG AGTACATCTA TGGAGGAATT ACGCCTGCTC AAAATAATAA AGTAAATCAT
AAATTATTGG GAAATCTATT TATTTCGGGA GAATCTCAAC AGAACTTAAA TAACAAGATT
ATTCTAGAAA AGGATATCGT AACTTTCCAG GAAATTGACT TTAAAATCAG AAAATACCTT
ATGGATAATT ATAAAATTTA TGACGCTACT TCTCCTTATG TAAGCGGCAG AATCGAAATT
GGCACAAAAG ATGGGAAACA TGAGCAAATA GACTTATTTG ACTCACCAAA TGAAGGGACT
AGATCAGATA TTTTTGCAAA ATATAAAGAT AATAGAATTA TCAATATGAA GAACTTTAGT
CATTTCGATA TTTATCTTGA A

### Protein Sequence - wild type

DSKKDISNVK SDLLYAYTIT PYDYKDCRVN FSTTHTLNID TQKYRGKDYY ISSEMSYEAS
QKFKRDDHVD VFGLFYILNS HTGEYIYGGI TPAQNNKVNH KLLGNLFISG ESQQNLNNKI
ILEKDIVTFQ EIDFKIRKYL MDNYKIYDAT SPYVSGRIEI GTKDGKHEQI DLFDSPNEGT
RSDIFAKYKD NRIINMKNFS HFDIYLE

### SPEC- Y15A.C27S.N79C.R181Q

GACTCTAAGA AAGACATTTC GAATGTTAAA AGTGATTTACT TTATGCATA CACTATAACT
GATTTACT TTGTGCATA CAC
C27S
CCTTATGATT ATAAAGATTG CAGGGTAAAT TTTTCAACGAC ACACACATT AAACATTGAT
GATT ATAAAGATTC CAGGGTAA
ACTCAAAAAT ATAGAGGGAA AGACTATTAT ATTAGTTCCGA AATGTCTTA TGAGGCCTCT
N79C
CAAAAATTTA AACGAGATGA TCATGTAGAT GTTTTTGGATT ATTTTATAT TCTTAATTCT
ATAT TCTTTGTTCT
CACACCGGTG AGTACATCTA TGGAGGAATT ACGCCTGCTCA AAATAATAA AGTAAATCAT
CA
AAATTATTGG GAAATCTATT TATTTCGGGA GAATCTCAACA GAACTTAAA TAACAAAATT

ATTCTAGAAA AAGATATCGT AACTTTCCAG GAAATTGACT TTAAAATCAG AAAATACCTT

ATGGATAATT ATAAAATTTA TGACGCTACT TCTCCTTATG TAAGCGGCAG AATCGAAATT

GGCACAAAAG ATGGGAAACA TGAGCAAATA GACTTATTTG ACTCACCAAA TGAAGGGACT
CAAGGGACT
R181Q
AGATCAGATA TTTTTGCAAA ATATAAAGAT AATAGAATTA TCAATATGAA GAACTTTAGT
CAATCAGATA TTTTTGC

CATTTCGATA TTTATCTTGAA

### Protein Sequence (combined mutants)

DSKKDISNVK SDLLAAYTIT PYDYKDSRVN FSTTHTLNID TQKYRGKDYY ISSEMSYEAS
QKFKRDDHVD VFGLFYILCS HTGEYIYGGI TPAQNNKVNH KLLGNLFISG ESQQNLNNKI
ILEKDIVTFQ EIDFKIRKYL MDNYKIYDAT SPYVSGRIEI GTKDGKHEQI DLFDSPNEGT
QSDIFAKYKD NRIINMKNFS HFDIYLE

### Example 4: Purification of recombinant wild-type and mutant proteins

Recombinant wild-type or mutant superantigens are expressed in *E*. *coli*. Two commercial vectors pGEX-2T (Pharmacia) and pET32A (New England Biolab) have been modified to introduce a new proteolytic cleavage site between the fusion protein and the superantigen. Separation of the two halves of the fusion protein is accomplished with the highly specific 3C protease that only cleaves at the single recognition site.
Two methods are currently used to purify fusion proteins.
a. pGEX-2T produces a fusion protein with the N-terminal component as the Glutathione S-Transferase linked to the superantigen sequence through a protein linker that contains a 3C-protease cleavage site. The fusion protein is purified from the crude bacterial lysate in single step purification on glutathione agarose. Fusion protein is eluted from the glutathione agarose with a buffer containing 5mM glutathione and cleaved by the addition of recombinant 3C protease. Superantigen is further purified by ion exchange HPLC chromatography.
b. pET32-A-3C. Protein is expressed as a stable thioredoxin fusion protein with a 6 histidine tag allowing single-step purification by metal chelation chromatography. Separation of the thioredoxin from superantigen is achieved by cleavage with recombinant 3C protease followed by HPLC ion exchange chromatography.

### Expression and purification of the recombinant protein

E.coli transformants are grown overnight at 37°C in a small 100 ml starter culture of Luria Broth (LB) containing 50 mg/ml ampicillin. A 1 litre culture is seeded in the morning and grown to mid-log phase, when IPTG is added to 0.1 mM to induce expression of the fusion protein. The culture is continued for 3 hours at which time cells are pelleted by centrifugation and disrupted by a combination of lysozyme and sonication. The clarified lysate is passed over either a 5 ml GSH agarose column or a Ni-NTA column. After thorough washing, bound protein is eluted by either 5 mM GSH (GSH agarose) or a buffer containing imidazole (MC chromatography).

The fusion protein is cleaved overnight at room temperature by recombinant 3C protease at a ratio of 1:500 (i.e. 2 mg 3C protease to 1 mg fusion protein). Superantigen is separated from fusion protein by two rounds of cation exchange chromatography. Protein is filter sterilised and stored at 1 mg/ml at 4°C until required.

### Introduction of disulphide coupling sites into SPE-C

An exposed cysteine residue has been introduced into the N-terminus of a TcR negative SPE-C at position N79. N79 is located within the putative TcR binding site. Several positions were tested before a residue was identified that met the following criteria
a. Surface exposed and accessible
b. Displayed efficient coupling of synthetic peptide
c. Did not interfere with MHC class II binding
d. Did not render the resulting SAG:peptide conjugate insoluble.
In addition to the introduced cysteine, a naturally occurring cysteine residue at position 27 was mutated to serine to avoid complications with refolding and interference with coupling.

The mutant of SPE-C used herein to provide examples of in vitro and *in vivo* immunomodluatory activity is SPEC-Y15A.C27S.N79C.R181Q, which is a composite of all mutations so far described above that abrogates TcR binding (Y15A and R181Q), introduce an efficient coupling residue (N79C) and removes a naturally occuring cysteine which interfered with coupling (C27S)

### Example 5: A truncated version SPEC lacking the N-terminal domain

In addition to the SPEC- SPEC-Y15A.C27S.N79C, an SPEC truncated mutant has been developed by deleting residues 22-90 (SPEC(-20-90))from the wild-type sequence This removes the entire TcR binding region plus the small N-terminal domain. This truncated mutant expresses very well in *E*. *coli,* is soluble and retains MHC class II binding activity. A cysteine residue has beenintroduced at position 92 to effect antigen coupling using the same method as described for the full length SPEC-Y15A.C27S.N79C molecule. The importance of this mutant is that it is much smaller, less antigenic (less likely to promote anti-SPEC antibody responses), and will be entirely devoid of any TcR binding ability. It is most unlikely that this truncated SPEC will have any toxicity effects *in vivo that are normally associated with wild-type toxins*.

The primary nucleotide sequence of truncated version of SPE-C is detailed below:

### DNA sequence (Factor X sequence shown in gray):

GAC TCT AAG AAA GAC ATT TCG AAT GTT AAA AGT GAT TTA CTT TGC GCA TAC ACT
ATA ACT CCT ATC GAA GGT CGT ACG CCT GCT CAA AAT AAT AAA GTA AAT CAT AAA
TTA TTG GGA AAT CTA TTT ATT TCG GGA GAA TCT CAA CAG AAC TTA AAT AAC AAG
ATT ATT CTA GAA AAG GAT ACC GTA ACT TTC CAG GAA ATT GAC TTT AAA ATC AGA
AAA TAC CTT ATG GAT AAT TAT AAA ATT TAT GAC GCT ACT TCT CCT TAT GTA AGC
GGC AGA ATC GAA ATT GGC ACA AAA GAT GGA AAA CAT GAG CAA ATA GAC TTA TTT
GAC TCA CCA AAT GAA GGG ACT AGA TCA GAT ATT TTT GCA AAA TAT AAA GAT AAT
AGA ATT ATC AAT ATG AAG AAC TTT AGT CAT TTC GAT ATT TAT CTT GAA AAA TAA

### Protein Sequence

D S K K D I S N V K S D L L C A Y T I T P I E G R T P A Q N N K V N H K L
L G N L F I S G E S Q Q N L N N K I I L E K D T V T F Q E I D F K I R K Y
L M D N Y K I Y D A T S P Y V S G R I E I G T K D G K H E Q I D L F D S P
N E G T R S D I F A K Y K D N R I I N M K N F S H F D I Y L E K Stop

### Example 6: TcR binding defective versions of SMEZ and SEA

In addition to SPE-C, TcR binding mutants of both SMEZ and SEA using site directed mutagenesis have been prepared. Comparative data of mutant vs wild-types on T cell proliferation is presented in table 3.

**Table 3. SMEZ mutants defective in TcR binding**

| Mutant | P50% (pg/ml) | Reduction |
|---|---|---|
| SMEZ -2 wild type | 2.0 | |
| SMEZ-2 W75L | >10 ng/ml | >100,000 |
| SMEZ-2 D42N | 10 ng/ml | 10,000 |
| SMEZ-2 W75L.D42N.K182Q | >10 ng/ml | >100,000 |

The aim was to produce mutants which stimulate T cells at, for example, about 0.0001 % of the activity of the wild type SAG. In addition, a cysteine residues is introduced in the same position relative to N79 in SPE-C.

Including two other superantigens is important to determine whether enhancement of immunogenicity is a feature of all superantigens, or specific to SPE-C. It is clearly broadly applicable, using the principles and techniques described herein.

Similar truncation mutants can be made for other superantigens such as SEA and SMEZ, using the methodology employed for the SPE-C mutants and the information on the Tcell receptor binding regions of the SAGs already published (for example reference #4, incorporated herein by reference).

### Example 7: Peptide coupling procedure

Both protein and peptide are stored in 10 mM phosphate pH6.0 under nitrogen to prevent oxidation and auto-dimerisation through the free cysteine.

Synthetic peptide containing a C-terminal cysteine residue and SPEC-Y15A.C27S.N79C are mixed together and incubated at room temperature for 1 hour at a molar ratio of 1:2 in a alkaline buffer containing 1 µM Cu²⁺. The copper acts as a redox catalyst. In the example below, a synthetic peptide of the pigeon cytochrome C (PCC) is provided, but this method will work for other peptides also so long as a free sulphur atom is present in the peptide.

| SPEC-Y15A.C27S.N79C.R181Q (MW 26,500) 10 mg/ml (380 mM) | PCC peptide (RADLIAYLKQATKC) (MW 1400) 10 mg/ml (700 mM) | Buffer |
|---|---|---|
| 100 µl | 10 µl | 200mM Tris pH8.0, 1 µM CuSO₄ |

Routinely >80% of SPEC-Y15A.C27S.N79C.R181 is shown to couple to peptide in a ratio of 1:1 Efficiency of coupling is assessed by SDS polyacrylamide gel electrophoresis. The SPEC-Y15A.C27S.N79C:peptide conjugate has a slower mobility on SDS PAGE consistent with an increase in molecular weight from the addition of a single peptide. Addition of 1 mM dithiothreitol (DTT) to the conjugate prior to SDS PAGE increases the electrophoretic mobility consistent with a reduction in molecular weight . This indicates that peptide coupling is via a reversible disulphide bond formation - a feature deemed important for dissociation of peptide once inside the APC.

### Example 8: Testing of responses to SAG:peptide conjugates

### The 5C. C7 T cell Receptor transgenic mouse

This mouse was obtained from The Malaghan Institute for Medical Research, Wellington School of Medicine, Mein St Wellington South, New Zealand

These mice were first generated by Berg et al (Ref 17).

The 5C.C7 transgenic mouse was originally constructed by Berg et al. ¹⁷. This mouse is transgenic for a TcR specific for the pigeon cytochrome C (PCC) peptide presented by mouse I-A^{d}. Greater than 80% of mature T cells from 5C.C7 mice express the transgenic TcR and respond to synthetic PCC peptide RADLIAYLKQATK *in vitro.* This mouse provides an excellent means to test PCC specific T cell responses both *in vitro* and *in vivo* as well as conduct adoptive transfer experiments. Adoptive transfer is a powerful method that allows the introduction of PCC reactive T cells into non-transgenic mice to study responses at varying T cell precursor frequencies.

### Antigenicity of SA G:PCC peptide to 5C. C7 T cells

This experiment determines how potent the SAG:peptide conjugate is *in vitro*. It is a test of how well the antigen is taken up and presented by the APCs present in culture and whether the binding of SAG to MHC class II enhances presentation to T cells.

Lymph node T cells from adult 5C.C7 mice were incubated with varying amounts of either synthetic PCC peptide alone, SPEC-Y15A.C27S.N79C, PCC peptide and SPEC-Y15A.C27S.N79C.R181 unconjugated or conjugated prior to addition in culture. MHC class II restricted T cell responses were measured by a 3-day ³H thymidine incorporation assay. Methods used were standard techniques such as those described Current Protocols in Immunology (1998) Colligan, J., Kuisbeck, A.M. Shevach, E.M. and W. Strober eds. John Wiley & Sons, Inc (ref 25)

### Results

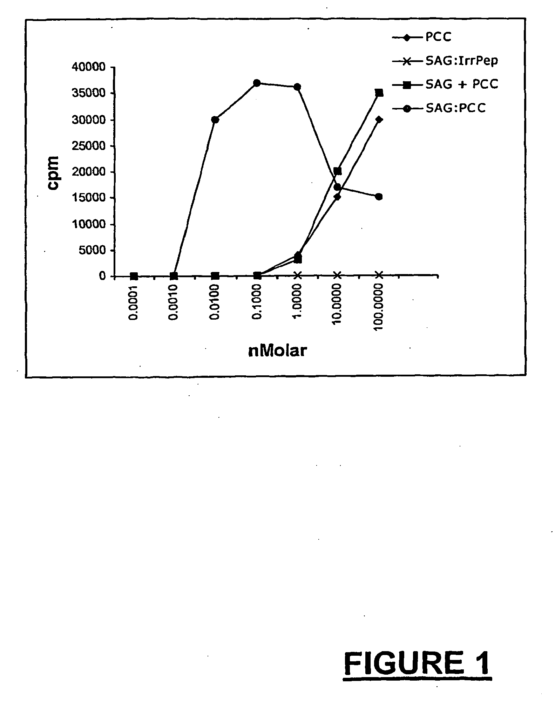

Fig. 1 indicates that 5C.C7 T cells responded to 10,000 times less SAG:PCC conjugate than the peptide alone. Optimal response to the SAG:PCC conjugate occurred at 10pM compared to 100 nM for the same components added in unconjugated form. No response was observed to SAG: irrelevant peptide indicating that the response was specific to the PCC peptide.

### Immunogenicity of SAG:PCC conjugate in 5C.C7 mice

This tests the ability of the SAG:peptide conjugate to generate an immune response *in vivo* and is a test of it's immunogenicity - that is to stimulate and expand peptide specific T cells.

### (i) Adoptive transfer of 5C.C7 T cells into wild-type C57BI/6 mice

Normal female C57BI/6 recipient mice receive 5 x 10⁶ 5C.C7 lymph node cells IP 1 week prior to immunisation.

### Immunisation protocol

Antigens were injected as a single subcutaneously (SC) dose as a stable emulsion with Freund's incomplete adjuvant in mature female C57BI/6 mice that had previously received 5C.C7 T cells. Two mice were injected for each dose with one of:
1. PCC peptide alone (1 and 100 mg)
2. PCC peptide + SPEC-Y15A.C27S.N79C.R181
3. SPEC:PCC conjugate (20 ng)

Mice were sacrificed 10 days later and the draining mesenteric lymph nodes removed. 1 x 10⁵ lymph node cells/well were cultured in duplicate with varying amounts of synthetic PCC peptide and the proliferative response of T cells measured by the 3 day ³H thymidine incorporation assay.

### Results

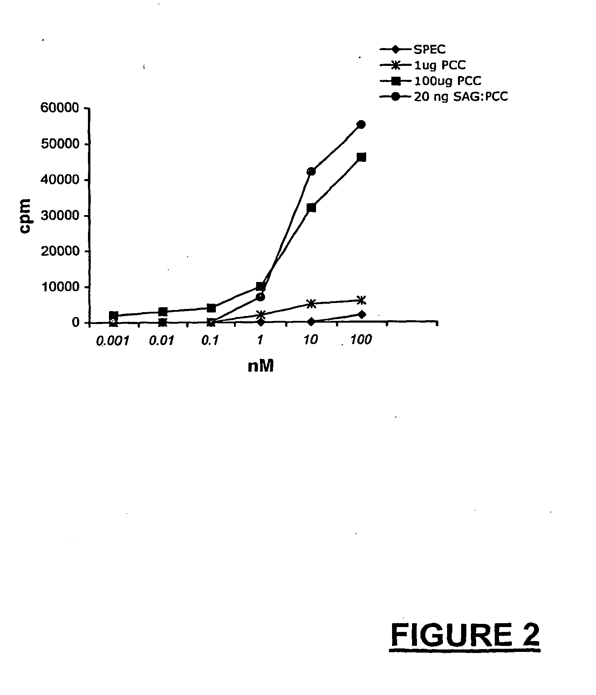

Figure 2 indicates that the lowest dose of SAG:PCC conjugate used to immunised 5C.C7 mouse was 20 ng and this produced optimal immunity equivalent to 100 mg of free PCC peptide. 1 mg of PCC peptide was non immunogenic. Thus the SAG:PCC conjugate was at least 10,000 times more immunogenic than free peptide. Irrelevant peptides coupled to SPEC generated no detectable immune response. It is likely that even lower doses of SAG:PCC conjugate will be immunogenic, increasing the effective difference in potency between conjugated and unconjugated PCC peptide to 100,000 times.

These studies show that SPEC-Y15A.C27S.N79C.R181 acts as an efficient delivery vehicle for poorly immunogenic antigens such as synthetic peptides. Not only is the peptide significantly more antigenic *in vitro,* but this also translates into enhanced immunogenicity *in vivo*. The immunogenicity of the PCC peptide increased by at least 10,000 times by coupling to the TcR binding defective superantigen SPEC-Y15A.C27S.N79C.

SPE-C mutant defective in MHC class II binding does not enhance antigenicity of the PCC peptide.

A recombinant mutant of SPE-C was created that disrupts the single zinc binding site to MHC class II. This mutant was coupled to synthetic PCC peptide and tested for its ability to stimulate 5C.C7 T cells *in vitro* compared to normal SPEC:PCC conjugate.

The results show that the mutant SPEC:PCC conjugate was no more antigenic than the SPEC + free peptide alone. This indicates that enhanced antigenicity is a result of SPE-C's ability to bind to cells expressing MHC class II, a function unique to superantigens.

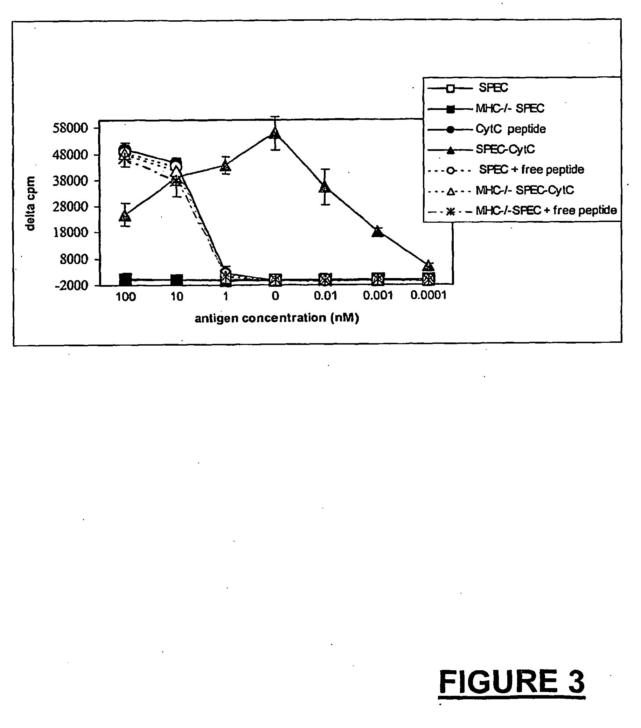

Figure 3 shows data which reveals the importance of MHC class II binding to enhancement of antigenicity and that SPEC is not simply acting as a "nonspecific" carrier protein.

### Example 10: Coupling of multiple peptides

Coupling need not be limited to individual peptides. Because immune responses to peptides are tightly restricted by the MHC polymorphisms of the host, it might be appropriate in some circumstances, to immunise with sets of peptides to generate broad spectrum immunomodulatory agents. Multiple peptides representing various components of a larger antigen such as a virus, bacteria or other protein antigen may be coupled by procedures described above or modified versions therefore which would be clear to those skilled in the art, to provide a mixed peptide:SAG conjugate antigen response to increase the diversity of the conjugate. Moreover, the ratio of peptides could be easily controlled to fine tune the immune response to a more desired outcome.

In further embodiments of the present invention, and applying the principles described herein, the following can also be accomplished:
- MHC class I and class II restricted peptides may be combined to provide improved helper CD4 and cytolytic CD8 effector cells.
- Immunodominant peptides from more than one viral antigen may be combined to promote selective anti-viral immunity.
- Peptides from regions of viral antigens that do not normally predominate in the protective immune response but represent regions of the virus essential to its replication or life cycle and are by nature strongly conserved may be used. This is particularly important in developing vaccines against highly mutating viruses such as retroviruses (e.g. HIV).
- Peptides and other antigens can be combined together and delivered by the immunomodulators to enhance or modulate the immune response.

### Example 11: Coupling of larger antigens and complex structures

Polypeptides and proteins can be coupled using the same procedures described above by reversible disulphide interchange to mutant SAGs. In addition, larger structures such as viruses can be "coated" with a TcR defective SAG by first treating the virus with a chemical that introduces a reactive sulphydryl group.

If the polypeptide has a naturally occurring exposed cysteine residues, coupling may be achieved to SAG directly without the need to introduce a reactive sulphydryl group. In this case, coupling would follow the established procedure outlined above.

### Chemical coupling methods

If the polypeptide does not have a naturally occurring cysteine, there are two methods that introduced a reactive sulphydryl group
a. A cysteine residue can be introduced genetically into the recombinant peptide and the polypeptide expressed from a heterologous expression system (prokaryotic or eukaryotic)
b. A chemical coupling reagent can be employed to introduce a reactive sulphydryl into the target protein or larger structure. A number of chemicals can be employed to introduce reactive sulphur groups onto proteins and other structures. One such chemical is N-succinimidyl S-acetylthiolproprionate (SATA - Piece Chemicals) and its close analogue SATP. This chemical converts a free amino groups on a protein or larger structure to a protected sulphydryl group which is activated with hydroxylamine. This allows coupling of other sulphydryl containing proteins such as SPEC-Y15A.C27S.N79C.R181via a reducible disulphide bond.
Relevant techniques are described in Ref. 21, incorporated herein by reference.

Delivery of proteins known to generate protective immunity for a particular pathogen can be made more immunogenic by first conjugating the protein to a TcR ablated SAG. The polypeptide would be broken down internally by the APC to present multiple restricted peptide epitopes to the host immune system. Anti-viral immunity might be enhanced by adding on molecules that selectively target the virus to APCs such as dendritic cells.

### Example 12: Multiple Sclerosis and EAE in mice

For multiple sclerosis, the predominant self antigen appears to be the Myelin Basic Protein (MBP) which is the major component of the myelin sheath. Experimental Allergic Encephalitis (EAE) is a well-established mouse model for the human disease multiple sclerosis. EAE can be generated by immunising susceptible mice with myelin basic protein (MBP) which produces anti-MBP reactive T cells that attack the myelin coating of nerves, leading to the encephalitic disease characterised by loss of motor control ¹⁸.

The EAE model can be used to examine the ability of mutant SAG:MBP peptides or mutant SAG:MBP protein conjugates to inhibit the start of the disease, or to suppress existing disease ¹⁹. Peptides (both agonist and antagonist) from the myelin basic protein (MBP) will be tested for their ability to suppress the onset of the EAE disease in mice.

### Example 13: Anti-viral responses and MHC class I restricted peptides

Mutant SAG:peptide conjugates could also serve to enhance MHC class I restricted CTL responses. CD8 positive CTL recognise peptides presented by MHC class I derived from viral infection and replication via the endogenous processing pathway. It has been shown however that there is significant crosstalk between the endogenous and exogenous pathway for peptides to be "shared" by both MHC class I and MHC class II molecules.

Protective cytolytic responses against viral infection or tumours are believed to require an obligate CD4 MHC class II dependent response as well as MHC class I restricted CD8 responses to provide long lasting protective immunity. Thus vaccines constructed from the conjugation of MHC class I restricted peptides and SAG mutants or a combination of both MHC class I and MHC class II restricted peptides would offer a flexible approach to designing efficient vaccines which promote both CD4 and CD8 responses.

### The LCMV₃₃₋₄₁ peptide and the 318 transgenic mice

The 318 transgenic mouse is a C57BU6 mouse with a transgenic TcR which recognises the lymphocyte choriomeningitis virus (LCMV) peptide in the context of the MHC class I antigen H-2D^{b} 20. The sequence of the active peptide is CKAVYNFATM which originates from the nucleocapsid protein. The 318 mouse will be used to model the ability of SPEC-Y15A.C27S.N79C.R181and other TcR defective SAGs to deliver MHC class I restricted peptides to CD8 cytotoxic T cells. Efficiency of delivery will be measured by the amount of SAG:LCMV conjugate required to generate a cytotoxic response against target cells pre-incubated with LCMV peptide (standard cytotoxic assay).

### The ⁵¹Cr release cytotoxicity assay to measure MHC class I restricted responses

Target cells (P814) are incubated with ⁵¹Cr and pulsed with LCMV peptide for 1 hour at 37°C. Cells are washed by centrifugation and mixed with lymph node cells from immunise mice at varying E:T ratios.

Cells are centrifuged lightly and incubated at 37°C for 1 hour. Supernatant is removed and counted for ⁵¹Cr to determine the degree of cell lysis.
Synthetic LCMV peptide modified at position 8 (M8C) will be coupled to SPEC-Y15A.C27S.N79C.R181 using the same method as described above. SAG:LCMV will be used to determine the *in vitro* response in lymph node cells from 318 mice.

### Resistance to viral infection

Mice infected with LCMV succumb within 14 days to the cytopathic effects. Mice immunised against LCMV develop a CTL response which provides full protection against. Mice immunised with SAG:LCMV will be tested for their resistance to wild-type LCMV virus.

### Example 14: Anti-tumour immunity

Many novel cancer immunotherapies attempt to break host tumour tolerance by targeting potential tumour specific antigens (usually lineage specific or differentiation antigens) directly to dendritic cells. We will test the hypothesis that TcR defective SAGs might usefully target tumour specific antigens to APCs and promote costimulatory signals that enhance antigen presentation. Initial studies will employ a tumour model in the 318 TcR transgenic mouse.

### The Lewis Lung carcinoma and the 318 transgenic mouse

We will initially employ a mouse model of tumour protection using the 318 transgenic mouse. A Lewis Lung carcinoma cell line transfected with a gene expressing the LCMV glycoprotein provides a model to investigate the ability of 318 mice to reject tumours. This cell line has high metastatic potential. Mice will be immunised with SAG:LCMV peptide and then inoculated with tumour cells. The degree of metastatic foci will be established at varying time points following inoculation and compared with non-immunised mice.

Mice will also be inoculated and then immunised at varying time points following tumour inoculation to determine whether immunisation protects established tumour growth.

### Example 15: Increasing the antigenicity of a whole protein to T cells by coupling to SAG.

1 mg whole Pigeon Cytochrome C protein (PCC) (Sigma) was treated with 1 mg of the cross-linked reagent N-succinimidyl S-acetylthioproprionate (SATP)(Pierce) for 1 hour at room temperature at pH7.0. Excess cross-linker was removed by gel chromatography using well established protocols, and the PCC-SATP activated with 0.1 M hydroxylamine and incubated with 100 µg recombinant SAG for 1 hour at pH8.5 to allow the proteins to couple. Conjugate was separated from free reactants by size exclusion chromatography according to well established protocols.

This method results in approximately 30% of the SAG forming a conjugate with PCC in a molar ratio of 1:1.

Conjugates were incubated with cultures of lymph node cells from T cells 5C-C7 mice and proliferation of T cells measured by ³H thymidine incorporation after 3 days, according to well established protocols. Results of these studies are shown in Figure 5.

The results show a substantial increase in the antigenicity towards PCC protein when conjugated to either SPEC or SMEZ. They further emphasises the importance of binding of the SAG to MHC class II to achieve increased antigenicity.

Some of the advantages and features of the exemplary TcR defective immunomodulatory conjugates of the present invention are the following:
a. The SAG is totally defective in binding to all TcRs and thus will be non-toxic in vivo.
b. Coupling of peptides is simple, efficient and reversible and broadly applicable.
c. The SAG:peptide conjugate is soluble.
d. SAG binding to MHC class II enhances APC activation of immunogenic and non-immunogenic moieties.

Although the present invention has been described with reference to certain preferred embodiments it will be understood that variations, which are in keeping with the broad principles and the spirit of the invention, are also contemplated to be within its scope.

### REFERENCES

### A. Staphylococcal superantigens

SEA Betley MJ, Mekalanos JJ (1988). Nucleotide sequence of the type A staphylococcal enterotoxin gene. J Bacteriol 170(1):34-41. Huang IY, Hughes JL, Bergdoll MS, Schantz EJ (1987). Complete amino acid sequence of staphylococcal enterotoxin A. J Biol Chem. 262(15):7006-13.
**SEB** Jones CL, Khan SA (1986). Nucleotide sequence of the enterotoxin B gene from Staphylococcus aureus. J Bacteriol. 166(1):29-33.
**SEC1** Bohach GA, Schlievert PM (1987). Nucleotide sequence of the staphylococcal enterotoxin C1 gene and relatedness to other pyrogenic toxins. Mol Gen Genet. 209(1):15-20.
**SEC2** Bohach GA, Schlievert PM (1989). Conservation of the biologically active portions of staphylococcal enterotoxins C1 and C2. Infect Immun. 57(7):2249-52.
**SEC3** Hovde CJ, Hackett SP, Bohach GA (1990). Nucleotide sequence of the staphylococcal enterotoxin C3 gene: sequence comparison of all three type C staphylococcal enterotoxins. Mol Gen Genet. 220(2):329-33.
**SED** Bayles KW, landolo JJ. 1989. Genetic and molecular analyses of the gene encoding staphylococcal enterotoxin D. J Bacteriol. 171(9):4799-806.
**SEE** Couch JL, Soltis MT, Betley MJ (1988). Cloning and nucleotide sequence of the type E staphylococcal enterotoxin gene. J Bacteriol. 170(7):2954-60.
**SEG** Munson SH, Tremaine MT, Betley MJ, Welch RA. 1998. Identification and characterization of staphylococcal enterotoxin types G and I from Staphylococcus aureus. Infect Immun. 66(7):3337-4.8.
**SEH** Ren K, Bannan JD, Pancholi V, Cheung AL, Robbins JC, Fischetti VA, Zabriskie JB. 1994. Characterization and biological properties of a new staphylococcal exotoxin. J Exp Med. 180(5):1675-83.
**SEI** Munson SH, Tremaine MT, Betley MJ, Welch RA. 1998. Identification and characterization of staphylococcal enterotoxin types G and I from Staphylococcus aureus. Infect Immun. 66(7):3337-48.
**SEJ** Zhang,S., landolo,J.J. and Stewart,G.C. 1998. The enterotoxin D plasmid of Staphylococcus aureus encodes a second enterotoxin determinant (sej). FEMS Microbiol. Letters 168; 227-233.
**TSST** Blomster-Hautamaa DA, Kreiswirth BN, Kornblum JS, Novick RP, Schlievert PM. 1989. The nucleotide and partial amino acid sequence of toxic shock syndrome toxin-1. J Biol Chem. 261 (33):15783-6.

### B. Streptococcal superantigens.

**SpeA** Johnson LP, L'ltalien JJ, Schlievert PM. 1986. Streptococcal pyrogenic exotoxin type A (scarlet fever toxin) is related to Staphylococcus aureus enterotoxin B. Mol Gen Genet. 203(2):354-6.
**SpeB** Hauser AR, Schlievert PM. 1990. Nucleotide sequence of the streptococcal pyrogenic exotoxin type B gene and relationship between the toxin and the streptococcal proteinase precursor.J Bacteriol. 172(8):4536-42.
**SpeC** Goshorn SC, Schlievert PM. 1988. Nucleotide sequence of streptococcal pyrogenic exotoxin type C. Infect Immun. 56(9):2518-20.
**SpeF** Norrby-Teglund A, Newton D, Kotb M, Holm SE, Norgren M. 1994. Superantigenic properties of the group A streptococcal exotoxin SpeF (MF). Infect Immun. 62(12):5227-33.
**SpeG** Proft T, Moffatt SL, Berkahn CJ, Fraser JD. 1999. Identification and characterization of novel superantigens from Streptococcus pyogenes. J Exp Med. 189(1):89-102.
**SpeH** Proft T, Moffatt SL, Berkahn CJ, Fraser JD. 1999. Identification and characterization of novel superantigens from Streptococcus pyogenes. J Exp Mend. 189(1):89-102.
**Spel** McLaughlin R.L., Sezate, S., Ferretti J.J. 1999. Molecular Characterization of Genes Encoding SPE-H and SPE-1. XIV. LISSSD.
**SpeJ** Proft T, Moffatt SL, Berkahn CJ, Fraser JD. 1999. Identification and characterization of novel superantigens from Streptococcus pyogenes. J Exp Med. 189(1):89-102.
**SSA** Mollick JA, Miller GG, Musser JM, Cook RG, Grossman D, Rich RR. 1993. A novel superantigen isolated from pathogenic strains of Streptococcus pyogenes with aminoterminal homology to staphylococcal enterotoxins B and C. J Clin Invest. 92(2):710-9.
**SMEZ** Kamezawa Y, Nakahara T, Nakano S, Abe Y, Nozaki-Renard J, Isono T. 1997. Streptococcal mitogenic exotoxin Z, a novel acidic superantigenic toxin produced by a T1 strain of Streptococcus pyogenes. Infect Immun. Sep;65(9):3828-33.
**SMEZ-2** Proft T, Moffatt SL, Berkahn CJ, Fraser JD. 1999. Identification and characterization of novel superantigens from Streptococcus pyogenes. J Exp Med. Jan 4;189(1):89-102.
**SMEZ-3 - SMEZ-24** Proft T, Moffatt SL, Weller KD, Paterson A, Martin D, Fraser JD. 2000. The streptococcal superantigen SMEZ exhibits wide allelic variation, mosaic structure, and significant antigenic variation. J Exp Med. 15;191(10):1765-76.

### General References

1. Kotzin, B. L., Leung, D. Y., Kappler, J. & Marrack, P. Superantigens and their potential role in human disease. Adv Immunol 54, 99-166 (1993).
2. Marrack, P. & Kappler, J. The Staphylococcal enterotoxins and their relatives. Science 248, 705-711 (1990).
3. Fraser, J. D., Arcus, V., Kong, P., Baker, E. N. & Proft, T. P. Superantigens - powerful modifiers of the immune system. Molecular Medicine Today 6, 125-135 (2000).
4. Li, H., Liera, A. & Mariuzza, R. A. Structure-function studies of T-cell receptor-superantigen interactions. [Review] [52 refs]. Immunological Reviews 163, 177-86 (1998).
5. Hudson, K. R. et al. Staphylococcal enterotoxin A has two cooperative binding sites on major histocompatibility complex class II. J. Exp. Med. 182, 711-20 (1995).
6. Li, P. L., Tiedemann, R. E., Moffat, S. L. & Fraser, J. D. The superantigen streptococcal pyrogenic exotoxin C (SPE-C) exhibits a novel mode of action. Journal of Experimental Medicine 186, 375-83 (1997).
7. Jardetzky, T. S. et al. Three-dimensional structure of a human class II histocompatibility molecule complexed with superantigen. Nature 368, 711-8 Issn: 0028-0836 (1994).
8. Banchereau, J. et al. Immunobiology of dendritic cells [Review]. Annual Review of Immunology 18 (2000).
9. Banchereau, J. & Steinman, R. M. DENDRITIC CELLS AND THE CONTROL OF IMMUNITY [Review]. Nature 392, 245-252 (1998).
10. Tiedemann, R. E. & Fraser, J. D. Cross-linking of MHC class II molecules by staphylococcal enterotoxin A is essential for antigen-presenting cell and T cell activation. Journal of Immunology 157, 3958-66 (1996).
11. Mehindate, K. et al. Cross-Linking Of Major Histocompatibility Complex Class Ii Molecules By Staphylococcal Enterotoxin a Superantigen Is a Requirement For Inflammatory Cytokine Gene Expression. Journal of Experimental Medicine 182, 1573-1577 (1995).
12. Marrack, P., Blackman, M., Kushnir, E. & Kappler, J. The toxicity of staphylococcal enterotoxin B in mice is mediated by T cells. J Exp Med 171, 455-64 (1990).
13. Fields, B. A. et al. Crystal structure of a T-cell receptor beta-chain complexed with a superantigen [see comments]. Nature 384, 188-92 (1996).
14. Irwin, M. J., Hudson, K. R., Fraser, J. D. & Gascoigne, N. R. Enterotoxin residues determining T-cell receptor V beta binding specificity. Nature 359, 841-3 (1992).
15. Acharya, K. R. et al. Structural basis of superantigen action inferred from crystal structure of toxic-shock syndrome toxin-1. Nature 367, 94-7 (1994).
16. Earhart, C. A. et al. STRUCTURES OF FIVE MUTANTS OF TOXIC SHOCK SYNDROME TOXIN-1 WITH REDUCED BIOLOGICAL ACTIVITY. Biochemistry 37, 7194-7202 (1998).
17. Berg, L. J. et al. Expression of T-cell receptor alpha-chain genes in transgenic mice. Molecular & Cellular Biology 8, 5459-69 (1988).
18. Wucherpfennig, K. W. & Strominger, J. L. Molecular mimicry in T cell-mediated autoimmunity: viral peptides activate human T cell clones specific for myelin basic protein. Cell 80, 695-705 (1995).
19. Brocke, S. et al. Induction of relapsing paralysis in experimental autoimmune encephalomyelitis by bacterial superantigen. Nature 365, 642-4 (1993).
20. Pircher, H., Burki, K., Lang, R., Hengartner, H. & Zinkernagel, R. M. Tolerance induction in double specific T-cell receptor transgenic mice varies with antigen. Nature 342, 559-61 (1989).
21. Duncan, R.J.S., Weston, P.D., Wrigglesworth, R. (1983) A new reaent which may be used to introduce sulphydryl groups into proteins, and itsuse in the preparation of conjugates for immunoassay. Anal. Biochem. 132, 68-73.
22. Sundberg, J. and J. Porath (1974) Attachment of group-containing ligands to insolbule polymers by means of bifunctional oxiranes. J. of Chromatography 90, 87-98.
23. Simons, P. and D.L. Vnder Jagt (1977) Purification of Glutathione-S-Transferease from human liver by glutathione-affinity chromatography. Anal. Biochem. 82 334-34
24. Ho, SN, Hunt HD, Horton RM, Pullen JK, Pease LR (1989) Site directed mutagenesis by overlap ectension using the polymerase chain reaction. Gene 67:31-40.
25. Current Protocols in Immunology (1998) Coligan, J, Kruisbeck A.M., Margulies D.H., Shevach, E., and W. Strober. Eds John Wiley & Sons NY.
26. Roussel A, Baker HM, Fraser JD, Baker EN (1997) Crystal structure of the streptococcal superantigen SPE-C: dimerisation and zinc binding suggests a novel mode of interaction with MHC class II molecules NATURE STRUCTURAL BIOLOGY. 4(8): 635-643

## Claims

1. An immunomodulator which comprises a superantigen coupled to an antigen, wherein said superantigen includes a Class II MHC binding site and a T-cell receptor binding site of a superantigen, the T-cell receptor binding site having one or more mutations that remove or minimize TcR binding compared to the wild type T-cell receptor binding site, and wherein in use the immunomodulator can bind Class II MHC molecules.

2. An immunomodulator according to claim 1, wherein the mutation of the T-cell receptor binding site is a substitution, deletion or addition.

3. An immunomodulator according to claim 1 or claim 2, wherein the T-cell receptor binding site of the superantigen has been deleted.

4. An immunomodulator according to any one of claims 1 to 3, wherein the superantigen is derived from *Staphylococcus aureus* and/or *Streptococcus pyogenes*.

5. An immunomodulator according to claim 4, wherein the superantigen is derived from SPE-C, SMEZ and/or SEA.

6. An immunomodulator according to any one of claims 1 to 5, wherein the superantigen is SPEC-Y15A or SPEC-Y15A R181Q or SPEC Y15A. C27S.N79C.R181 Q.

7. An immunomodulator according to any one of claims 1 to 6, wherein the superantigen is coupled reversibly to an antigen.

8. An immunomodulator according to any one of claims 1 to 7, wherein the antigen is a protein, a polypeptide and/or a peptide, or a nucleic acid.

9. An immunomodulator according to any one of claims 1 to 8, wherein the antigen is non-immunogenic when not coupled to the superantigen.

10. An immunomodulator according to any one of claims 4 or 7 to 9, wherein the superantigen is SPEC (-20-90).

11. Pharmaceutical composition comprising an immunomodulator according to any one of claims 1 to 10 and a pharmaceutically acceptable carrier, adjuvant, excipient and/or solvent.

12. Vaccine comprising an immunomodulator according to any one of claims 1 to 10.

13. An immunomodulator according to claims 1 to 10, a pharmaceutical composition according to claim 11, or a vaccine according to claim 12 for use in treating a disorder which requires the induction or stimulation of the immune system.

14. An immunomodulator, a pharmaceutical composition or a vaccine according to claim 13, wherein the disorder is selected from the group consisting of bacterial, viral, fungal or parasitic infection, autoimmunity, allergy and/or pre-neoplastic or neoplastic transformation.

15. Use of an immunomodulator according to any one of claims 1 to 10 for the preparation of a medicament for the therapeutic or prophylactic treatment of a disorder which requires the induction or stimulation of the immune system.

16. Use according to claim 15, wherein the disorder is selected from the group consisting of bacterial, viral, fungal or parasitic infection, autoimmunity, allergy and/or pre-neoplastic or neoplastic transformation.

17. Method of preparing an immunomodulator according to claim 1 comprising the steps of:
a) providing superantigen which includes a Class II MHC binding site and a T-cell receptor binding site of a superantigen,
b) introducing one or more mutation into the T-cell binding site of the superantigen, and
c) coupling an antigen to the superantigen.

18. A method according to claim 17, wherein the superantigen is selected from the group of SPE-C, SMEZ and SEA.

19. A method according to claim 18 wherein the superantigen is SPE-C and the mutation is Y15A R181Q, Y15A. C27S. N79C. R181Q, or (-20-90).

20. Method of increasing antigenicity of a compound, comprising the coupling of said compound to superantigens to produce the immunomodulator of claim 1, wherein said superantigen includes a Class II MHC binding site and a T-cell receptor binding site of a superantigen, the T-cell receptor binding site having one or more mutations that remove or minimize TcR binding compared to the wild-type T-cell receptor binding site.

21. A method according to claim 20, wherein the mutation of the T-cell receptor binding site is a substitution, deletion or addition.

22. A method according to claim 20 or claim 21, wherein the T-cell binding site of the superantigen has been deleted.

23. A method according to any one of claims 20 to 22, wherein the superantigen is derived from *Staphylococcus aureus* and/or *Streptococcus pyogenes*.

24. A method according to claim 23, wherein superantigen is derived from SPE-C, SMEZ and/or SEA.

25. A method according to claim 24, wherein the superantigen is SPEC-Y15A or SPEC-Y15A R181Q or SPEC-Y15A. C27S. N79C. R181Q or SPEC (-20-90).

26. A method according to any one of claims 20 to 25, wherein the superantigen is coupled reversibly to said compound.

27. A method according to any one of claims 20 to 26, wherein the compound is selected from the group consisting of a protein, a polypeptide and/or a peptide, a carbohydrate or a nucleic acid.

28. A method according to any one of claims 20 to 27, wherein the compound is non-immunogenic when not coupled to the superantigen.

29. An immunomodulator according to any one of claims 1 to 10, wherein the mutated T-cell receptor binding site removes or minimizes TcR binding to equal to or greater than 10,000 fold compared to the wild-type T-cell receptor binding site.

## Patentansprüche

1. Immunmodulator, der ein Superantigen umfasst, das an ein Antigen gebunden ist, wobei das Superantigen eine MHC-Klasse-II-Bindungsstelle und eine T-Zellen-Rezeptor-Bindungsstelle eines Superantigens enthält, wobei die T-Zellen-Rezeptor-Bindungsstelle eine oder mehrere Mutationen aufweist, die die TcR-Bindung im Vergleich zur Wildtyp-T-Zellen-Rezeptor-Bindungsstelle entfernt oder minimiert, und wobei der Immunmodulator in Verwendung MHC-Klasse-II-Moleküle binden kann.

2. Immunmodulator nach Anspruch 1, wobei die Mutation der T-Zellen-Rezeptor-Bindungsstelle eine Substitution, Deletion oder Addition ist.

3. Immunmodulator nach Anspruch 1 oder 2, wobei die T-Zellen-Rezeptor-Bindungsstelle des Superantigens deletiert ist.

4. Immunmodulator nach einem der Ansprüche 1 bis 3, wobei das Superantigen von *Staphylococcus aureus* und/oder *Streptococcus pyogenes* abgeleitet ist.

5. Immunmodulator nach Anspruch 4, wobei das Superantigen von SPE-C, SMEZ und/oder SEA abgeleitet ist.

6. Immunmodulator nach einem der Ansprüche 1 bis 5, wobei das Superantigen SPEC-Y15A oder SPEC-Y15A R181Q oder SPEC Y15A. C27S. N79C. R181Q ist.

7. Immunmodulator nach einem der Ansprüche 1 bis 6, wobei das Superantigen reversibel an ein Antigen gebunden ist.

8. Immunmodulator nach einem der Ansprüche 1 bis 7, wobei das Antigen ein Protein, ein Polypeptid und/oder ein Peptid oder eine Nukleinsäure ist.

9. Immunmodulator nach einem der Ansprüche 1 bis 8, wobei das Antigen nicht-immunogen ist, wenn es nicht an das Superantigen gebunden ist.

10. Immunmodulator nach einem der Ansprüche 4 oder 7 bis 9, wobei das Superantigen SPEC (-20-90) ist.

11. Pharmazeutische Zusammensetzung, umfassend einen Immunmodulator nach einem der Ansprüche 1 bis 10 und einen pharmazeutisch akzeptablen Träger, ein pharmazeutisch akzeptables Adjuvans, einen pharmazeutisch akzeptablen Exzipienten und/oder ein pharmazeutisch akztables Lösungsmittel.

12. Vakzin, umfassend einen Immunmodulator nach einem der Ansprüche 1 bis 10.

13. Immunmodulator nach den Ansprüchen 1 bis 10, pharmazeutische Zusammensetzung nach Anspruch 11 oder Vakzin nach Anspruch 12 zur Verwendung bei der Behandlung einer Störung, die der Induktion oder Stimulation des Immunsystems bedarf.

14. Immunmodulator, pharmazeutische Zusammensetzung oder Vakzin nach Anspruch 13, wobei die Störung aus der Gruppe bestehend aus bakterieller Infektion, viraler Infektion, Pilz- oder Parasiteninfektion, Autoimmunität, Allergie und/oder preneoplastischer oder neoplastischer Transformation ausgewählt ist.

15. Verwendung eines Immunmodulators nach einem der Ansprüche 1 bis 10 zur Herstellung eines Medikaments zur therapeutischen oder prophylaktischen Behandlung einer Störung, die der Induktion oder Stimulation des Immunsystems bedarf.

16. Verwendung nach Anspruch 15, wobei die Störung aus der Gruppe bestehend aus bakterieller Infektion, viraler Infektion, Pilz- oder Parasiteninfektion, Autoimmunität, Allergie und/oder preneoplastischer oder neoplastischer Transformation ausgewählt ist.

17. Verfahren zur Herstellung eines Immunmodulators nach Anspruch 1, umfassend die Schritte:
a) Bereitstellen eines Superantigens, das eine MHC-Klasse-II-Bindungsstelle und eine T-Zellen-Rezeptor-Bindungsstelle eines Superantigens enthält,
b) Einbringen einer oder mehrerer Mutationen in die T-Zellen-Rezeptor-Bindungsstelle des Superantigens, und
c) Binden eines Antigens an das Superantigen.

18. Verfahren nach Anspruch 17, wobei das Superantigen aus der Gruppe von SPE-C, SMEZ und SEA ausgewählt ist.

19. Verfahren nach Anspruch 18, wobei das Superantigen SPE-C und die Mutation Y15A R181Q, Y15A. C27S. N79C. R181Q oder (-20-90) ist.

20. Verfahren zum Erhöhen der Antigenität einer Verbindung, umfassend das Binden der Verbindung an Superantigene, um den Immunmodulator nach Anspruch 1 herzustellen, wobei das Superantigen eine MHC-Klasse-II-Bindungsstelle und eine T-Zellen-Rezeptor-Bindungsstelle eines Superantigens enthält, wobei die T-Zellen-Rezeptor-Bindungsstelle eine oder mehrere Mutationen aufweist, die die TcR-Bindung im Vergleich zur Wildtyp-T-Zellen-Rezeptor-Bindungsstelle entfernt oder minimiert.

21. Verfahren nach Anspruch 20, wobei die Mutation der T-Zellen-Rezeptor-Bindungsstelle eine Substitution, Deletion oder Addition ist.

22. Verfahren nach Anspruch 20 oder 21, wobei die T-Zellen-Rezeptor-Bindungsstelle des Superantigens deletiert ist.

23. Verfahren nach einem der Ansprüche 20 bis 22, wobei das Superantigen von *Staphylococcus aureus* und/oder *Streptococcus pyo*genes abgeleitet ist.

24. Verfahren nach Anspruch 23, wobei das Superantigen von SPE-C, SMEZ und/oder SEA abgeleitet ist.

25. Verfahren nach Anspruch 24, wobei das Superantigen SPEC-Y15A oder SPEC-Y15A R181Q oder SPEC Y15A. C27S. N79C. R181Q oder SPEC (-20-90) ist.

26. Verfahren nach einem der Ansprüche 20 bis 25, wobei das Superantigen reversibel an die Verbindung gebunden ist.

27. Verfahren nach einem der Ansprüche 20 bis 26, wobei die Verbindung aus der Gruppe bestehend aus Protein, Polypeptid und/oder Peptid, kohlehydrat oder Nukleinsäure ausgewählt ist.

28. Verfahren nach einem der Ansprüche 20 bis 27, wobei die Verbindung nicht-immunogen ist, wenn sie nicht an das Superantigen gebunden ist.

29. Verfahren nach einem der Ansprüche 1 bis 10, wobei die mutierte T-Zellen-Rezeptor-Bindungsstelle die TcR-Bindung im Vergleich zur Wildtyp-T-Zellen-Rezeptor-Bindungsstelle entfernt oder auf größer gleich das 10.000-Fache minimiert.

## Revendications

1. Immunomodulateur qui comprend un super-antigène couplé à un antigène, ledit super-antigène comprenant un site de liaison MHC de classe II et un site de liaison au récepteur des lymphocytes T d'un super-antigène, le site de liaison au récepteur des lymphocytes T ayant une ou plusieurs mutations qui éliminent ou minimisent la liaison de TcR par rapport au site de liaison au récepteur des lymphocytes T de type sauvage, et dans lequel, lors de l'utilisation, l'immunomodulateur peut se lier à des molécules MHC de classe II.

2. Immunomodulateur selon la revendication 1, dans lequel la mutation du site de liaison au récepteur des lymphocytes T est une substitution, une délétion ou une addition.

3. Immunomodulateur selon la revendication 1 ou la revendication 2, dans lequel le site de liaison au récepteur des lymphocytes T du super-antigène a été supprimé.

4. Immunomodulateur selon l'une quelconque des revendications 1 à 3, dans lequel le super-antigène est dérivé de Staphylococcus aureus et/ou de Streptococcus pyogenes.

5. Immunomodulateur selon la revendication 4, dans lequel le super-antigène est dérivé de SPE-C, SMEZ et/ou SEA.

6. Immunomodulateur selon l'une quelconque des revendications 1 à 5, dans lequel le super-antigène est SPEC-Y15A ou SPEC-Y15A R181Q ou SPEC-Y15A.C27S.N79C.R181 Q.

7. Immunomodulateur selon l'une quelconque des revendications 1 à 6, dans lequel le super-antigène est couplé de façon réversible à un antigène.

8. Immunomodulateur selon l'une quelconque des revendications 1 à 7, dans lequel l'antigène est une protéine, un polypeptide et/ou un peptide, ou un acide nucléique.

9. Immunomodulateur selon l'une quelconque des revendications 1 à 8, dans lequel l'antigène est non immunogène lorsqu'il n'est pas couplé au super-antigène.

10. Immunomodulateur selon l'une quelconque des revendications 4 ou 7 à 9, dans lequel le super-antigène est SPEC (-20-90).

11. Composition pharmaceutique comprenant un immunomodulateur selon l'une quelconque des revendications 1 à 10 et véhicule, adjuvant, excipient et/ou solvant pharmaceutiquement acceptable.

12. Vaccin comprenant un immunomodulateur selon l'une quelconque des revendications 1 à 10.

13. Immunomodulateur selon les revendications 1 à 10, composition pharmaceutique selon la revendication 11 ou vaccin selon la revendication 12, pour l'utilisation dans le traitement d'un trouble qui nécessite l'induction ou la stimulation du système immunitaire.

14. Immunomodulateur, composition pharmaceutique ou vaccin selon la revendication 13, où le trouble est choisi dans le groupe comprenant une infection bactérienne, virale, fongique ou parasitaire, une auto-immunité, une allergie et/ou une transformation pré-néoplasique ou néoplasique.

15. Utilisation d'un immunomodulateur selon l'une quelconque des revendications 1 à 10, pour la préparation d'un médicament pour le traitement thérapeutique ou prophylactique d'un trouble qui nécessite l'induction ou la stimulation du système immunitaire.

16. Utilisation selon la revendication 15, dans laquelle le trouble est choisi dans le groupe comprenant une infection bactérienne, virale, fongique ou parasitaire, une auto-immunité, une allergie et/ou une transformation pré-néoplasique ou néoplasique.

17. Procédé de préparation d'un immunomodulateur selon la revendication 1, comprenant les étapes consistant à :
a) fournir un super-antigène qui comprend un site de liaison MHC de classe II et un site de liaison au récepteur des lymphocytes T d'un super-antigène,
b) introduire une ou plusieurs mutations dans le site de liaison aux lymphocytes T du super-antigène, et
c) coupler un antigène au super-antigène.

18. Procédé selon la revendication 17, dans lequel le super-antigène est choisi dans le groupe comprenant SPE-C, SMEZ et SEA.

19. Procédé selon la revendication 18, dans lequel le super-antigène est SPE-C et la mutation est Y15A R181Q, Y15A.C27S.N79C.R181Q ou (-20-90).

20. Procédé d'augmentation de l'antigénicité d'un composé, comprenant les couplages dudit composé aux super-antigènes pour produire l'immunomodulateur selon la revendication 1, dans lequel ledit super-antigène comprend un site de liaison MHC de classe II et un site de liaison au récepteur des lymphocytes T d'un super-antigène, le site de liaison au récepteur des lymphocytes T ayant une ou plusieurs mutations qui éliminent ou minimisent la liaison de TcR par rapport au site de liaison au récepteur des lymphocytes T de type sauvage.

21. Procédé selon la revendication 20, dans lequel la mutation du site de liaison au récepteur des lymphocytes T est une substitution, une délétion ou une addition.

22. Procédé selon la revendication 20 ou la revendication 21, dans lequel le site de liaison au récepteur des lymphocytes T du super-antigène a été supprimé.

23. Procédé selon l'une quelconque des revendications 20 à 22, dans lequel le super-antigène est dérivé de Staphylococcus aureus et/ou de Streptococcus pyogenes.

24. Procédé selon la revendication 23, dans lequel le super-antigène est dérivé de SPE-C, SMEZ et/ou SEA.

25. Procédé selon la revendication 24, dans lequel le super-antigène est SPEC-Y15A ou SPEC-Y15A R181Q ou SPEC-Y15A.C27S.N79C.R181Q ou SPEC (-20-90).

26. Procédé selon l'une quelconque des revendications 20 à 25, dans lequel le super-antigène est couplé de façon réversible audit composé.

27. Procédé selon l'une quelconque des revendications 20 à 26, dans lequel le composé est choisi dans le groupe comprenant une protéine, un polypeptide et/ou un peptide, un glucide ou un acide nucléique.

28. Procédé selon l'une quelconque des revendications 20 à 27, dans lequel l'antigène est non immunogène lorsqu'il n'est pas couplé au super-antigène.

29. Immunomodulateur selon l'une quelconque des revendications 1 à 10, dans lequel le site de liaison au récepteur des lymphocytes T élimine ou minimise la liaison de TcR à un niveau supérieur ou égal à un facteur 10 000 par rapport au site de liaison au récepteur des lymphocytes T de type sauvage.
